Europäisches Patentamt

**European Patent Office**

**Office européen des brevets**

(19)

(11) Publication number: **0 172 361**
A2

(12) # EUROPEAN PATENT APPLICATION

(21) Application number: **85107916.0**

(22) Date of filing: **26.06.85**

(51) Int. Cl.⁴: **C 12 N 15/00**
C 12 P 21/02, C 12 N 1/20
//(C12N1/20, C12R1:19)

(30) Priority: **29.06.84 US 626219**

(43) Date of publication of application:
**26.02.86 Bulletin 86/9**

(84) Designated Contracting States:
**CH DE FR GB IT LI**

(71) Applicant: **QUEEN'S UNIVERSITY AT KINGSTON**

**Kingston Ontario K7L 3N6(CA)**

(72) Inventor: **Davies, Peter L.**
**23 Michael Grass Crescent**
**Kingston, Ontario(CA)**

(74) Representative: **Sajda, Wolf Eckart, Dipl.-Phys. et al,**
**MEISSNER, BOLTE & PARTNER Postfach 86 06 24**
**D-8000 München 86(DE)**

(54) **Cloned cardionatrin cDNA, cleaved cardionatrin cDNA and cardionatrin precursors, their preparation, corresponding plasmids and microorganisms comprising said plasmid.**

(57) A novel cloned cardionatrin cDNA is produced from rat atria. The cardionatrin cDNA codes for a pro-cardionatrin precursor, which contains cardionatrin 1-28 at its C-terminal end. Also provided are methods of making the cloned cardionatrin cDNA, pro-cardionatrin, a cleaved cDNA which codes exclusively for the cardionatrin 1-28, corresponding plasmids and transformed microorganisms.

EP 0 172 361 A2

Cloned cardionatrin cDNA, cleaved cardionatrin cDNA and
cardionatrin precursors, their preparation, corresponding
plasmids and microorganisms comprising said plasmid

This invention relates to the synthesis and isolation of cloned cardionatrin cDNA from rat atrial tissue, as well as to the cloned DNA itself and cardionatrin precursors. The invention further relates to a method of incorporating the cloned DNA into a plasmid, inserting the plasmid into a microorganism so that the microorganism produces a corresponding peptide from the inserted DNA, and extracting the peptide therefrom.

It is well established that mammalian atria have fluid volume regulating capacity. The atrial muscle of mammals, in contrast to the muscle of the ventricle, contains secretory-storage granules called specific atrial granules. These granules have morphological and histochemical properties in common with storage granules present in cells which store and synthesize polypeptide hormones. Significant changes occur in the number of atrial granules in the rat following alterations in electrolyte and water balance, and this has led to the view that these granules play a role in fluid volume regulation. It has been discovered that mammalian atrial extract, when injected into rats, causes natriuresis and diuresis. Natriuresis is an increased excretion of cations, particularly sodium, and diuresis is increased excretion of water. It has therefore been hypothesized that the secretory-like morphological features of mammalian atrial cardiocytes are related to the capacity of these cells to synthesize and store peptides which are potent natriuretic and diuretic agents.

Early purification attempts which were concerned with isolating these peptides from rat atrial muscle produced a mixture of biologically active peptides differing in molecular weights. These peptides were collectively referred to as atrial natriuretic factor (de Bold, A.J., Borenstein, H.B., Veress, A.T. and Sonnenberg, H., Life Sci. 28 (1981) 89-94, and de Bold, A.J., Proc. Soc. Exp. Biol. Med. 170 (1982) 133-138). The name cardionatrin was proposed to differentiate the individual peptides (de Bold, A.J. and Flynn, T.G., Life Sci. 33 (1983) 297-302). One of these peptides, rat cardionatrin, containing 28 amino acids has been sequenced (Flynn, T.G., de Bold, M. and de Bold, A.J., Biochem. Biophys. Res. Commun. 117 (1983) 859-865). Subsequently, the sequence of several rat atrial peptides which were truncated versions of the 28 amino acid cardionatrin were discovered (Currie, M.G., Geller, D.M., Cole, B.R., Siegel, N.R., Fok, K.F., Adams, S.P., Eubanks, S.R., Galluppi, G.R. and Needleman, P., Science 223 (1984) 67-69; Mison, K.S., Fukoni, H., Crammer, R.T. and Inagani, T., Biochem. Biophys. Res. Commun. 119 (1984) 524-529). Even more recently, the amino acid sequence of longer peptides, which contain the sequence of cardionatrin 1-28 at their C-termini have been reported (Thibault, G., Garcia, R., Cantin, M., Genest, J., Lazure, C., Seidah, N.G. and Chretien, M., FEBS Lett. 167 (1984) 352-356); Kangawa, K., Fukuda, A., Minanino, N. and Matsuo, H., Biochem. Biophys. Res. Commun. 119 (1984) 933-940). Finally, the sequence of atrial natriuretic factor from human heart has also been determined (Kangawa, K. and Matsuo, H., Biochem. Biophys. Res. Commun. 118 (1984) 131-139). The disclosure of the above identified publications is incorporated herein by reference.

The larger peptides isolated from the heart muscle have shown natriuretic and diuretic activity. This fact suggests that the biologically active peptide, for example cardionatrin 1-28, is produced by processing from a precursor molecule. Accordingly, there was a need in the art for methods for the synthesis of cardionatrin and related and precursor materials.

The objects of this invention are to provide

a cloned cardionatrin cDNA which codes for a precursor cardionatrin peptide,

a method for synthesizing and isolating it,

a precursor peptide for which the cloned cardionatrin cDNA codes,

a method for obtaining the precursor peptide from the cloned cardionatrin cDNA,

a cleaved, cloned cardionatrin cDNA, cleaved from the long chain cloned cardionatrin DNA, which codes for the cardionatrin 1-28 peptide,

a method for producing the cardionatrin 1-28 peptide from the cleaved, cloned cardionatrin cDNA,

a plasmid containing cloned cardionatrin cDNA, as well as a microorganism into which the plasmid containing cloned cardionatrin cDNA has been incorporated,

and

a plasmid containing cleaved, cloned cardionatrin cDNA as well as a microorganism into which the plasmid containing cleaved, cloned cardionatrin cDNA has been incorporated.

The above objects are achieved according to the claims. The dependent claims are related to preferred embodiments.

In accordance with the above objects, the present invention provides a cloned cardionatrin cDNA isolated from rat atrial tissue. The cDNA is produced by extracting RNA from powdered rat atria and ventricles, purifying the RNA, and recovering the poly(A)$^+$ RNA. From the poly(A)$^+$ RNA, double stranded cDNA is synthesized. The 3' ends of the double stranded cDNA are then extended with dGMP residues and inserted into a plasmid which had previously been tailed with dCMP residues. The plasmids are then used to transform E. coli to ampicillin resistance. Selected colonies are hybridized to cDNA made to atrial poly(A)$^+$ RNA. The same colonies are then hybridized to cDNA made to total ventricle RNA. The clones that hybridized preferentially to atrial cDNA are then end labeled with DNA polymerase and sequenced. Two clones, identified herein as car 1 and car 3, are thus sequenced. The sequence analysis showed that a DNA sequence, coding for the cardionatrin 1-28, is located at the C-terminal end of the car 3 DNA. The car 3 DNA was thus shown to code for part of the precursor of cardionatrin 1-28, pro-cardionatrin.

The invention further provides the pro-cardionatrin peptide translated from the car 3 DNA, as well as a method of producing the peptide. The car 3 DNA may be inserted into a plasmid which has been prepared for reception thereof. The plasmid may then be used to transform a microorganism to manufacture pro-cardionatrin peptide.

Alternately, the car 3 DNA may be cleaved to form the DNA which codes specifically for cardionatrin 1-28. The cleaved DNA may be inserted into a plasmid, which may then be used to transform a microorganism to produce cardionatrin 1-28 peptide.

Reference is now made to the pictures wherein picture 1 shows a filter paper on which colonies of E. coli containing DNA clones were hybridized to specific cDNAs. The top filter, bearing DNA from 70 colonies, was probed with $^{32}$P labeled ventricle cDNA. The duplicate filter below was probed with atrial cDNA. The positions of car 1 and car 3, as well as the control clone (A&V) which hybridized well to both probes, are identified on both filters.

Picture 2 shows the results of the Northern blot analysis. Total RNA from atria and ventricles was separated by electrophoresis and transferred to DBM paper. Identical blots were probed with nick-translated car 1, car 3, and a clone which hybridizes strongly to both atrial and ventricular cDNAs. The positions of migration of the rRNA (L), small rRNA (S) and 5.8S rRNA (F) are shown relative to the origin of the gel (O).

This invention provides a novel cloned cardionatrin cDNA which codes for pro-cardionatrin peptide. This cloned cardionatrin DNA has been given the designation car-3. The car-3 DNA was derived from material isolated from extracts of rat atria.

In the extraction procedure, atria and ventricles were obtained from rats, rinsed in saline solution, and frozen. The frozen tissue was mixed with solid $CO_2$ and pulverized in a grinder. The RNA was then extracted from the powdered material using guanidine thiocyanate and phenol, according to the procedure of Feramisco et al. (Feramisco, J.R., Helfman, D.M., Smart, J.E., Burridge, K. and Thomas, G.P., J. Biol. Chem. 257 (1982) 11024-11031). Glycogen was removed by centrifugation, and the poly(A)$^+$ RNA material was selected by passage through oligo (dT) cellulose.

The corresponding doubled-stranded cDNA was then synthesized from the poly(A)$^+$ RNA using the method of Wickens et al. (Wickens, M.P., Buell, G.N. and Schimke, R.T., J. Biol. Chem. 253 (1978) 2483-2495). The cDNA was treated with nuclease, and the 3' ends were extended with dGMP residues using terminal transferase and dGTP. The cDNA was then inserted into a plasmid which had previously been tailed with dCMP residues. Suitable plasmids include pUC9, pBR322 and pUC8. The recombinant plasmids were used to transform E. coli to ampicillin resistance. The colonies which showed ampicillin resistance were plated out in duplicate. One set was hybridized to cDNA made from atrial poly(A)$^+$ RNA. The other set was hybridized to cDNA made from ventricle RNA. The clones that hybridized preferentially to atrial cDNA were further characterized.

The DNA was end labeled and sequenced according to the procedure of Maxam and Gilbert, Methods Enzymol. 65 (1980) 499-560).

Clones, identified as car 1 and car 3, hybridized to atrial cDNA but not ventricle cDNA. The car 3 cDNA was shown to have the following sequence:

(C)<sub>n</sub>GAGAGATGGAGGTGCTCTCGGGCGCGGCCCCTGGGACCC
CTCCGATAGATCTGCCCTCTTGAAAAGCAAACTGAGGGCTCTGCTCGCTGGCCCT
CGGAGCCTGCGAAGGTCAAGCTGCTTCGGGGGTAGGATTGACAGGATTGGAGCCC
AGAGCGGACTAGGCTGCAACAGCTTCCGGTACCGAAGATAACAGCCAAATCTGCT
CGAGCAGATCGCAAAAGATCCCAAGCCCTTTCGGTGTGTCACACA(G)<sub>n</sub> .

The car 3 DNA codes for the cardionatrin precursor, pro-cardionatrin. The active cardionatrin portion 1-28, resides at the C-terminal end of the precursor pro-cardionatrin. The sequence of the pro-cardionatrin, shown in register with the car 3 DNA sequence, is as follows:

Car 3

. 20
ArgAspGlyGlyAlaLeuGlyArgGlyProTrp
(C)<sub>n</sub>GAGAGATGGAGGTGCTCTCGGGCGCGGCCCCTGG

30                                    40
AspProSerAspArgSerAlaLeuLeuLysSerLysLeuArgAlaLeuLeuAlaGlyPro
GACCCCTCCGATAGATCTGCCCTCTTGAAAAGCAAACTGAGGGCTCTGCTCGCTGGCCCT

↓                50                                    60
ArgSerLeuArgArgSerSerCysPheGlyGlyArgIleAspArgIleGlyAlaGlnSer
CGGAGCCTGCGAAGGTCAAGCTGCTTCGGGGGTAGGATTGACAGGATTGGAGCCCAGAGC

70                    ↓
GlyLeuGlyCysAsnSerPheArgTyrArgArg
GGACTAGGCTGCAACAGCTTCCGGTACCGAAGATAACAGCCAAATCTGCTCGAGCAGATC

GCAAAAGATCCCAAGCCCTTTCGGTGTGTCACACA(G)<sub>n</sub> .

The cardionatrin 1-28 peptide resides between the two arrows in the sequence shown above. The sequence shows that cardionatrin 1-28 is followed by two arginine residues at the very C-terminal end of the precursor. Directly following these two arginine residues is a termination signal. Although the primary translation product of car 3 would contain these two residues, they do not appear in any of the cardionatrins or cardionatrin-like peptides that have been characterized up to now. It is not yet known whether the removal of these two basic residues serves to activate or modulate any of the physiological effects of cardionatrin. Normally a disulfide (S-S) bridge will bond the Cys groups in the peptide. If it is not present naturally on cloning, it can be introduced by oxidation.

As described by Flynn et al, Biochem. Biophys. Res. Commun. 117, (1983) 859-865, the pro-cardionatrin is a 28 amino acid peptide with diuretic and natriuretic activity in which the primary structure is: H-Ser-Leu-Arg-Arg-Ser-Ser-Cys-Phe-Gly-Gly-Arg-Ile-Asp-

$$\boxed{\text{S}-\text{S}}$$

Arg-Ile-Gly-Ala-Gln-Ser-Gly-Leu-Gly-Cys-Asn-Ser-Phe-(Arg)-Tyr-OH. The biological activity of this peptide is identical to that of atrial natriuretic factor and cardionatrin I isolated from rat atria.

The isolated car 3 DNA may be used in the production of pro-cardionatrin peptides. In this procedure, the car 3 may be inserted into a plasmid which has been prepared to receive it by addition of poly(G) and poly(C) tails to the ends of the plasmid DNA at the addition site. The plasmid may then be inserted into a microorganism, particularly E. coli, to transform the organism. The product, pro-cardionatrin peptide, can then be recovered from the microorganism.

The cardionatrin thus produced has natriuretic effects when injected into rats either by reason of the cardionatrin 1-28 active sequence or as a precursor of the cardionatrin 1-28 sequence in the host.

The car 3 DNA may also be cleaved so that the remaining DNA sequence codes exclusively for the cardionatrin 1-28. The sequence can then be modified by adding linker oligonucleotides and inserting into a plasmid termed an expression vector. This plasmid can then be inserted into microorganism, particularly an E. coli, to transform the microorganism. The microorganism will then produce cardionatrin 1-28, which can be extracted therefrom. Cardionatrin 1-28 has been shown to have potent diuretic and natriuretic activity, and represents the most active compound of the atrial natriuretic factor.

The peptides of the invention may be employed as diuretics and natriuretics as discussed herein. For this purpose, the compounds are preferably combined with a pharmaceutically acceptable carrier for administration preferably by injection. Conventional adjuvants and carriers may be employed in combination with about 0,001 to 2,0 weight percent of the active compound. The compounds may be administered to animals or humans at dosages sufficient to be effective.

The following examples illustrate the invention.

In the examples, as in the remainder of the specification, parts are by weight unless otherwise indicated.

Example 1

Atria and ventricles were obtained from 225-250g male Sprague-Dawley rats, rinsed in ice-cold, phosphate-buffered saline (pH 7,4), and frozen immediately in liquid $N_2$. The frozen tissue was mixed with solid $CO_2$ and pulverized in a pre-cooled coffee grinder.

RNA was extracted from the powdered atria and ventricles using guanidine thiocyanate in combination with phenol according to the procedure of Feramisco et al., as cited above. In this procedure, powdered atria (5g) were added to 25 ml of 4M guanidine thiocyanate containing 50 µM Tns-HC1 (pH 7,6), 2% sodium lauryl sarcosinate, 1% ß-mercaptoethanol and 10 µM EDTA at 60°C with stirring. The mixture was subsequently extracted with phenol at 60°C, phenol/chloroform, and then chloroform. The aqueous fraction was recovered, and RNA was precipitated from it by addition of two volumes of ethanol at -20°C. The RNA was recovered by centrifugation and then treated with proteinase K (200 µg/ml) in the presence of 0,2%sodium dodecyl sulfate at 37°C. Phenol/chloroform and chloroform extractions were repeated on the digest, and the RNA was again recovered from the aqueous fraction by ethanol precipitation. Glycogen was removed by centrifugation at a relative centrifugal acceleration of 120 000 for 1 h. Poly(A)[+] RNA was selected from total atrial RNA by chromatography on oligo (dT)-cellulose (cf. Aviv, H. and Leder, P., Proc. Nat. Acad. Sci. USA 69 (1972) 1408-1412).

Double stranded cDNA was synthesized from the atrial poly(A)[+] RNA using the method of Wickens et al, as cited above. Specifically, the poly (A)[+] RNA was mixed with an oligo (dT)$_{10}$ primer which was then extended by the enzyme reverse transcriptase to form single-stranded DNA complementary to the mRNA. This synthesis was performed at 42°C in the presence of all four desoxynucleoside triphosphates. The reaction mixture was boiled, then cooled and supplemented with fresh desoxynuceloside triphosphates. The second strand of the cDNA was synthesized using DNA polymerase I at 15°C with the first strand of the cDNA serving as both template and primer. After treatment with S1 nuclease, the 3' ends of the double-stranded cDNA were extended with dGMP residues using terminal transferase and dGTP (quanidine triphosphate). The cDNA was inserted into the Pst 1 site of pUC9 which had been previously tailed with 20-30 dCMP complementary residues. The recombinant plasmids were inserted into E. coli and used to transform E. coli JM83 to ampicillin resistance. Colonies which remained white in the presence of x-galactose were plated out in duplicate. One set was hybridized to cDNA made to atrial poly(A)$^+$ RNA. The other set was hybridized to cDNA made to total ventricle RNA. Those clones that hybridized preferentially to atrial cDNA were further characterized.

Example 2

To identify sequences in the cDNA of Example 1, that are expressed in atria but not ventricles, 800 clones from the atrial cDNA library were screened by differential colony hybridization (Picture 1). Clones, such as car 1 and car 3, which hybridized to atrial cDNA but not to ventricle cDNA (cf. Picture 1), were screened a second time with the same probes. Only clones car 1 and car 3 hybridized specifically to atrial cDNA in both screening tests.

Example 3

A Northern blot analysis was conducted on the car 1 and car 3 clones of Example 2. Equal quantities of total RNA's from atria and ventricles were electrophoresed in adjacent lanes on an agarose gel in the presence of methyl mercury hydroxide and then blotted onto DBM paper. Plasmid preparations were made from car 1 and car 3, and from a control clone which hybridized strongly to both atrial and ventricular cDNAs. The plasmids were (nick-translated) labeled with p32 during repair synthesis, and hybridized separately to the Northern blots. The control plasmid hybridized with equal intensity to a 900 nucleotide-long sequence from the library, ,in the atrial and ventricular lanes, whereas both car 1 and car 3 hybridized specifically to a 1 000 nucleotide-long sequence which was present only in the atrial lane (Picture 2).

The Northern blot analysis confirmed that cardionatrin is produced in the atrial but not the ventricular chambers of rat hearts, and also showed that cardionatrin mRNA was 1 000 nucleotides long.

Example 4

The sequences of the car 1 and car 3 DNA were determined. The DNA was end-labeled using the Klenow fragment of E. coli DNA polymerase 1, and sequenced according to the procedure of Maxam and Gilbert, as cited above. The inserts in car 1 and car 3 were released from their plasmid vectors by double-digestion with Bam H1 and Hind III, and were estimated to be 100 bp and 300 bp long, respectively, by agarose gel electrophoresis. DNA sequence analysis using the procedure of Maxam and Gilbert subsequently showed that car 1 and car 3 contained 74 bp and 249 bp of cDNA sequence, respectively, flanked by the appropriate homopolymeric tails. When all possible reading frames of the car 3 insert were analyzed, one was found to match the amino acid sequence of cardionatrin 1-28 and its N-terminal extension. With respect to the latter 73-residue sequence, the car 3 clone encodes for amino acids 14 to 73, in which residues 46-73 correspond to cardionatrin 1-28. The DNA sequence shows that there are two arginine codons after residue 73 followed by a termination codon. This is followed in turn by 59 residues of 3' untranslated region.

Example 5

Car 3 cDNA is inserted into the plasmid pUC9, which is previously tailed with dGMP and dCMP residues. The recombinant plasmids are used to transform E. coli JM83 to ampicillin resistance. The ampicillin resistant E. coli are plated out on a separate medium. The E. coli are given sufficient amino acid nutrients to produce large amounts of pro-cardionatrin peptide. The peptides are then extracted from the E. coli.

Example 6

The car 3 cDNA is cleaved with restriction enzymes so that the remaining DNA codes exclusively for cardionatrin 1-28. The 3' ends of the double stranded DNA are extended with linker oligonucleotides by using DNA ligase. The linkers are then cut open with restriction enzyme, and the cDNA, now containing sticky ends generated by the enzyme, is inserted into an expression vector which is cleaved with the same enzyme. The resulting recombinant plasmids are used to transform E. coli JM83 to ampicillin resistance. Those colonies exhibiting ampicillin resistance are plated out on a separate medium. The E. coli are provided with enough amino acid nutrients to produce large quantities of cardionatrin 1-28 peptide. The cardionatrin 1-28 peptide is then extracted from the E. coli.

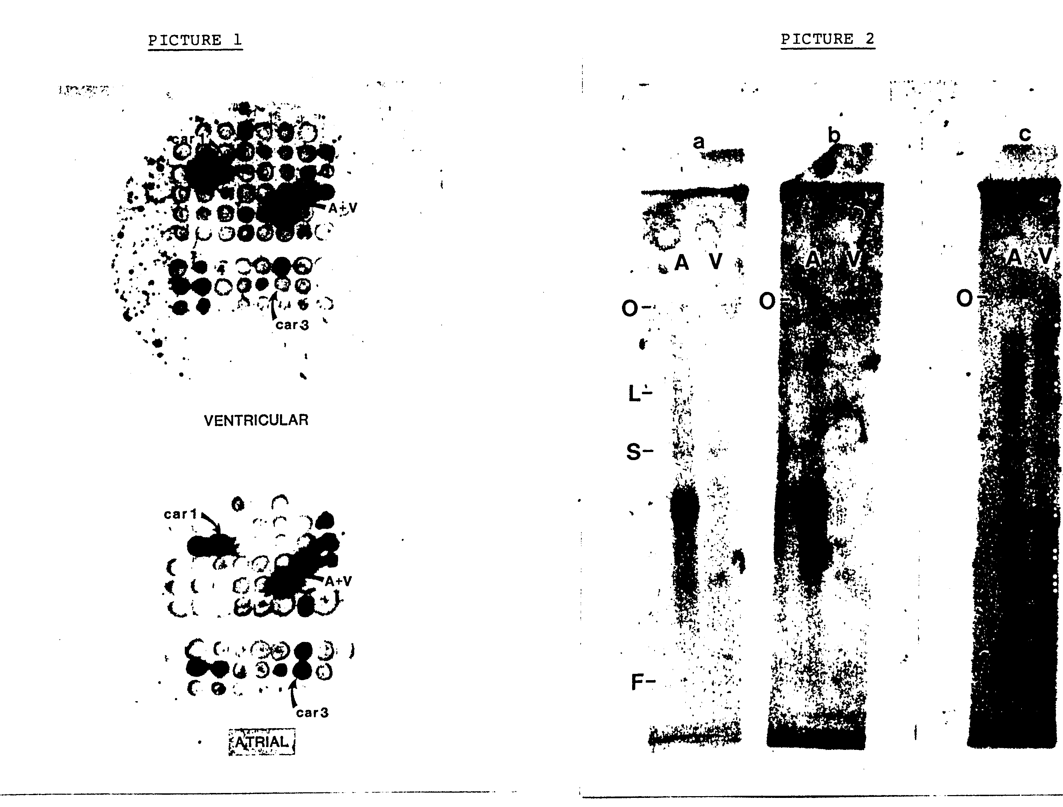

Claims

1.      Cloned cardionatrin cDNA, characterized by the codon sequence

$(C)_n$GAGAGATGGAGGTGCTCTCGGGCGCGGCCCCTGGGACCC
CTCCGATAGATCTGCCCTCTTGAAAAGCAAACTGAGGGCTCTGCTCGCTGGCCCT
CGGAGCCTGCGAAGGTCAAGCTGCTTCGGGGGTAGGATTGACAGGATTGGAGCCC
AGAGCGGACTAGGCTGCAACAGCTTCCGGTACCGAAGATAACAGCCAAATCTGCT
CGAGCAGATCGCAAAAGATCCCAAGCCCTTTCGGTGTGTCACACA$(G)_n$.

2.      The polypeptide translation product of the cDNA of claim 1, characterized by the sequence

ArgAspGlyGlyAlaLeuGlyArgGlyProTrpAspProSerAspArg
SerAlaLeuLeuLysSerLysLeuArgAlaLeuLeuAlaGlyProArgSerLeu
ArgArgSerSerCysPheGlyGlyArgIleAspArgIleGlyAlaGlnSerGly
LeuGlyCysAsnSerPheArgTyrArgArg .

3.      A method of producing the cDNA of cardionatrin, characterized by

isolating heart RNA from rats,

inserting the isolated RNA into a plasmid thereby forming a recombinant plasmid,

inserting the recombinant plasmid into a microorganism to transform the microorganism,

and testing the transformed microorganisms for characteristics of the cDNA.

4.      A method according to claim 3, characterized in that E. coli are used as said microorganisms.

5.      A method according to claim 3 or 4, characterized in that the characteristics of the cDNA are tested by hybridizing the transformed microorganisms to cDNA made from rat heart atria, as well as to cDNA made from rat heart ventricles, so that at least some transformed microorganisms hybridize preferentially to the atrial cDNA's, and selecting those transformed microorganisms which hybridize preferentially to the atrial cDNAs.

6.      A method according to any of claims 3 to 5, characterized in that pUC9 is used as said plasmid.

7.      A method of producing a coardionatrin polypeptide, characterized by

        incorporating a corresponding cloned cardionatrin cDNA into a plasmid,

        inserting the plasmid into a microorganism so that the microorganism is transformed and produces the polypeptide,

        and extracting the polypeptide therefrom.

8.      A method according to claim 7, characterized in that E. coli are used as said microorganisms.

9.    A method for producing a cleaved, cloned cDNA which codes only for cardionatrin from a cloned cardionatrin cDNA, characterized in that the cloned cardionatrin cDNA is cleaved with restriction enzymes.

10.    A method of producing cardionatrin from a cleaved cDNA, characterized by

        extending the 3' end of the cDNA with dGNP residues, forming a modified cDNA,

        inserting the modified cDNA into a plasmid which has been previously tailed with dCNP residues,

        inserting the plasmid into a microorganism, thereby transforming the microorganism,

        plating the transformed microorganism onto a fresh medium,

        providing the microorganism sufficient nutrients to produce the cardionatrin,

        and extracting the cardionatrin from the microorganism.

11.    A method according to claim 10, characterized in that E. coli are used as said microorganisms.

12.    Recombinant plasmids into which the cDNA of claim 1 has been inserted.

13.    Recombinant plasmids containing the cloned cardionatrin cDNA or the cleaved cardionatrin cDNA obtainable according to the method of any of claims 7 to 11.

14.    Microorganisms containing the recombinant plasmids of claim 12 or 13.

PICTURE 1

VENTRICULAR

ATRIAL

PICTURE 2

0172361